# EUROPÄISCHE PATENTSCHRIFT

(11) **EP 1 238 645 B1**
(45) Veröffentlichungstag und Bekanntmachung des Hinweises auf die Patenterteilung: **12.01.2005**
(21) Anmeldenummer: 01129077.2
(22) Anmeldetag: 07.12.2001
(51) Int. Cl.: A61K 7/06

(54) **Antischuppenmittel**
Antidandruff composition
Composition antipelliculaire

(30) Priorität: 09.03.2001 DE 10111288
(43) Veröffentlichungstag der Anmeldung: 11.09.2002
(73) Patentinhaber: Wella Aktiengesellschaft, 64274 Darmstadt (DE)
(72) Erfinder: Hehner, Ursula, 64395 Brensbach (DE); Ringert, Karl-Heinz, 64295 Darmstadt (DE); Hölzel, Hans, 64407 Fränkisch-Crumbach (DE)

(56) Entgegenhaltungen:
- EP-A- 0 680 745
- EP-A- 0 813 859
- WO-A-00/33807
- WO-A-92/14440
- WO-A-96/29983

## Beschreibung

Gegenstand der vorliegenden Erfindung ist ein Mittel mit reinigenden und pflegenden Eigenschaften enthaltend eine Kombination aus drei Antischuppenwirkstoffen bestehend aus Climbazole, Piroctone Olamine und Zinc Pyrithione sowie seine Verwendung bei Schuppenbildungen der haarbedeckten Haut, insbesondere der Kopfhaut.

Eine Vielzahl von Menschen leidet unter Schuppenbildung. Bereits das leichte Erscheinen von Schuppen auf der Kopfhaut und im Haar wird als Zeichen mangelnder Pflege angesehen. Auch der mit einer Schuppenbildung einhergehende Juckreiz der Haut wird als störend empfunden. Da Juckreiz häufig eine Kratzreaktion auslöst, kann es zusätzlich zu Verletzungen der betroffenen Hautareale kommen. Damit verbunden können als weitere Komplikationen Infektionen mit pathogenen Erregern auftreten.

Die in der Regel auf haarbedeckten Hautflächen, insbesondere der Kopfhaut, auftretende Schuppenbildung kann verschiedene Ursachen haben. Normalerweise entstehen Schuppen dadurch, dass die ständig nachwachsende oberste Hornschicht der Epidermis in Form kleinster Schuppen, den Mikroschuppen, abgestreift wird. Bei stark fettender Kopfhaut können Mikroschuppen durch den aus Talgdrüsen der Haarfollikel ausgeschiedenen Talg (Sebum) zu größeren Einheiten verkleben.

In vielen Fällen jedoch verursacht die mikrobielle Besiedlung der betroffenen Haut eine verfrühte und erhöhte Ablösung der obersten Hornschicht in Form von Schuppen. So ist zum Beispiel bekannt, dass der Hefepilz *Pityrosporum ovale* (syn. *Malassezia furfur*) die Kopfhaut von Schuppenträgern in signifikant höheren Dichten besiedelt als bei Personen ohne Schuppen (SCHRADER, K. In: Grundlagen und Rezepturen der Kosmetika, Hüthig (Hrsg.), 2. Auflage, 1989, Seite 970).

*Pityrosporum ovale* bewirkt durch Spaltung des Talges der Haut die Entstehung freier Fettsäuren und Lipoperoxiden und es kommt zur Reizung der Kopfhaut. Dadurch wird die Mitoseaktivität erhöht und es kommt zu einer vermehrten Korneozytenbildung, die eine erhöhte Schuppenbildung bewirkt. Bekannt ist, dass durch Hemmung von *Pityrosporum ovale* dieser Prozess erst gar nicht in Gang gesetzt wird und die Schuppenbildung ausbleibt.

Die Anforderungen an ein haarkosmetisches Mittel, welches die Schuppenbildung verhindern oder vermindern soll, sind vielseitig. Auf der einen Seite soll die Kopfhaut und damit auch das Haar anhaltend von Schuppen befreit werden. Andererseits soll das Mittel nicht nur für die Kopfhaut verträglich sein sondern auch das Haar soll bei Anwendung eines Antischuppenmittels nicht geschädigt werden und seinen Glanz und seine Kämmbarkeit behalten.

Es sind eine Reihe an haarkosmetischen Mitteln bekannt, die die Schuppenbildung der Kopfhaut verhindern oder vermindern sollen. Derartige kosmetische Mittel stehen entweder als Shampoos, Lotionen oder Haarwasser zur Verfügung.

Im Falle eines mikrobiellen Ursprungs der Schuppenbildung wurde die Anwendung von verschiedenen, speziellen Antischuppenwirkstoffen vorgeschlagen. Solche Antischuppenwirkstoffe sollen die Vermehrung der Mikroorganismen, insbesondere *Pityrosporum ovale,* auf der Kopfhaut hemmen.

Zu den bekannten Antischuppenwirkstoffen gehören die Verbindungen (1-(4-Chlorophenoxy)-1-(1H-imidazolyl)-3,3-dimethyl-2-butanone (INCI-Bezeichnung: Climbazole, Handelsname: Crinipan AD), (Bis(1-Hydroxy-2-(1H)-Pyridinethionato)Zink (INCI-Bezeichnung: Zinc Pyrithione, Handelsname: Zinc Omadine) sowie 1-Hydroxy-4-methyl-6-(2,4,4-trimethylpentyl)-2(1H)-pyridinone, 2-Aminoethanol Salz (INCI-Bezeichnung: Piroctone Olamine, Handelsname: Octopirox). Diese Verbindungen sind bekannt und kommerziell erhältlich. Hierzu kann zum Beispiel verwiesen werden auf "International Cosmetic Ingredient Dictionary and Handbook", Vol. 1 - 3, Seventh Edition 1997, The Cosmetic, Toiletry, and Fragrance Association, Washington DC; Schampoo zusammensetzungen, die Zinc Pyrithione, Piroctone Olamine oder/und Climbazole enthalten, sind aus WO 92/14440 A1, WO 00/33807 A1 und WO 96/29983 A1 bekannt. Darüber hinaus sind als weitere Antischuppenwirkstoffe solche aus der Gruppe synthetischer oder chemischer Verbindungen wie beispielsweise Salizylsäure, Schwefel, Teerpräparate, Undecensäurederivate und Hinokitiol und solche aus der Gruppe der Naturstoffe wie beispielsweise Extrakte aus Arnika, Birke, Klettenwurzel, Pappel, Brennessel oder Walnußschale bekannt.

Nachteilig ist jedoch, dass von den genannten Mitteln hohe Konzentrationen eingesetzt werden müssen, um einen schuppenmindernden oder schuppenverhindernden Effekt zu erzielen, wobei als häufige Begleiterscheinung unerwünschte Wirkungen an der Haut und dem Haar auftreten können.
Darüber hinaus besteht ein weiterer Nachteil darin, dass die einzelnen Wirkstoffe keine zufriedenstellende Wirkung erzielen. Selbst Kombinationen mit zwei Antischuppenwirkstoffen, beispielsweise Climbazole und Piroctone Olamine, die in Handelsprodukten verwendet werden, zeigen nicht immer eine zufriedenstellende schuppenmindernde oder schuppenverhindernde Wirkung.

Aufgabe der vorliegenden Erfindung war es daher, ein Mittel zur Verfügung zu stellen, welches die vorgenannten Nachteile nicht aufweist und sowohl eine verbesserte schuppenmindernde und schuppenverhindernde Wirkung einer bereits bestehenden Schuppenbildung als auch eine anhaltende vorbeugende Wirkung einer Schuppenbildung entfaltet.

Erfindungsgemäß wurde die Aufgabe gemäß Anspruch 1 gelöst, wonach ein Mittel bereitgestellt wird, enthaltend als Wirkstoffkombination die Verbindungen 1-(4-Chlorophenoxy)-1-(1H-imidazolyl)-3,3-dimethyl-2-butanone (INCI-Bezeichnung: Climbazole), Bis(1-Hydroxy-2-(1H)-Pyridinethionato)Zinc (INCI-Bezeichnung: Zinc Pyrithione) und 1-Hydroxy-4-methyl-6-(2,4,4-trimethylpentyl)-2(1H)-pyridinone, 2-Aminoethanol Salz (INCI-Bezeichung: Piroctone Olamine).
Überraschenderweise konnte nämlich festgestellt werden, dass ein Mittel gemäß Anspruch 1 und den weiteren Ausführungsformen gemäß den Unteransprüchen gegenüber dem bekannten Stand der Technik die Bildung von Schuppen besser verhütet oder vermindert oder eine bereits bestehende Schuppenbildung effektiver und nachhaltiger beseitigt oder lindert. Darüber hinaus konnte als weiterer Vorteil festgestellt werden, dass die verbesserte Wirkung sogar bei geringeren Mengen an Einzelwirkstoffen erzielt werden konnte. Dieser mit der erfindungsgemäßen Kombination erzielte Synergismus war nicht zu erwarten.

Das erfindungsgemäße Mittel kann vorteilhaft zur Verhütung der Schuppenbildung oder zur Behandlung (Beseitigung oder Linderung) einer bereits bestehenden Schuppenbildung der Kopfhaut oder anderer haarbedeckter Hautoberflächen (beispielsweise Bartregion, Achseln, Intimbereich) verwendet werden. Vorteilhaft ist dabei auch, dass es nach an sich bekannter Weise auf die Kopfhaut und auf die Kopfhaare bzw. auf die entsprechenden Körperstellen aufgetragen werden und nach relativ kurzer Einwirkzeit gegebenenfalls wieder leicht durch Ausspülen oder Abwaschen entfernt werden kann.
Erfindungsgemäß wird auch die Verwendung des erfindungsgemäßen Mittels zur Beseitigung, Minderung und Verhütung einer Schuppenbildung auf behaarten Körperoberflächen, wie insbesondere Kopfhaut, Bartregion, Achselhöhlen, Intimbereich, umfasst.

In vorteilhafter Weise kann der erfindungsgemäßen Kombination mindestens ein weiteres synthetisches chemisches und/oder natürliches Antischuppenmittel hinzugefügt werden.
Als synthetische chemische Antischuppenmittel kommen beispielsweise in Betracht: Salizylsäure, Schwefel, Teerpräparate, Undecensäurederivate, Hinokitiol, Antibiotika, Antimycotika. Als natürliche Antischuppenmittel sind Extrakte pflanzlichen Ursprungs zu nennen, beispielsweise Klettenwurzelextrakte, Pappelextrakte, Brennessel-extrakte, Walnußschalenextrakte, Birkenextrakte, Weidenrindenextrakte oder Arnikaextrakte.

Ein weiterer Gegenstand der vorliegenden Erfindung liegt in der kosmetischen Verwendung einer Kombination bestehend aus den Wirkstoffen Climbazole, Zinc Pyrithione und Piroctone Olamine zur Verhütung oder Verminderung der Schuppenbildung oder zur Beseitigung und Linderung einer bereits bestehenden Schuppenbildung auf behaarten Körperoberflächen, insbesondere auf der Kopfhaut, aber auch zur Herstellung einer Präparation für die Behandlung von Schuppen der Haut, wobei nicht nur die Behandlung bestehender Schuppen, sondern auch die vorbeugende, verhindernde Behandlung mit umfasst wird.
Anhand des an sich bekannten Plattendiffusionstests konnte mit dem Testorganismus *Pityrosporum ovale* nachgewiesen werden, dass mit der erfindungsgemäßen Kombination Climbazole plus Zinc Pyrithione plus Piroctone Olamine sich ein deutlich größerer Hemmhof ausbildet, als es bei den Einzelwirkstoffen oder bei der Kombination von zwei Wirkstoffen (Climbazole und Piroctone Olamine) der Fall war. Mit der erfindungsgemäßen Kombination ist demzufolge eine gegenüber den Einzelwirkstoffen und Zweierkombinationen stärkere Hemmwirkung einer Schuppenbildung zu erzielen. Darüber hinaus war es überraschend, dass die vergleichsweise stärkere Hemmwirkung mit Wirkstoffmengen erzielt werden konnte, die im Vergleich deutlich unter den Mengen der Einzelwirkstoffe oder einer Zweierkombination lagen.

Der mit dem Testorganismus *Pityrosporum ovale* durchgeführte Plattendiffusionstest ist an sich bekannt. Die folgende Beschreibung soll das Verfahren beispielhaft verdeutlichen:
Beim Testorganismus *Pityrosporum ovale syn. Malassezia furfur* (Stamm CBS 6000) wurde von einer lyophilisierten Kultur ausgegangen. Vor der Herstellung der Impfsuspension wurde lyophilisiertes Material in Sabouraud-Bouillon mit Olivenöl (Fluka Art. Nr. 75357, *Pityrosporum ovale* wächst nur mit Ölzusatz) vorkultiviert (48 Stunden, 42°C). Anschließend folgten zwei Passagen auf Sabouraud-4% Dextrose Agar (Merck Art. Nr.5438), auf dessen Oberfläche mit einem Wattestäbchen ein dünner Olivenölfilm aufgetragen wurde. Hierzu wurde aus der bewachsenen Bouillon mittels Wattestäbchen Material von *P. ovale* entnommen, auf die betreffenden Nährbodenplatten flächendeckend aufgetragen und für 48 Stunden bei 32°C inkubiert. Für die Herstellung der Impfsuspension wurde die Plattenkultur mit 5,0 ml Ringerlösung mit Zusatz von 0,1% Tween 80 (Merck Art. Nr. 822187) abgeschwemmt, mit einer 5 ml Pipette aufgenommen und in ein steriles Reagenzröhrchen pipettiert und gut homogenisiert. Am Fotometer wurde auf Extinktion 1,0 (620 nm) eingestellt. Die erhaltene Suspension wurde als Impfmaterial verwendet. 200 ml des genannten Nährmediums wurden mit 0,5 ml dieser Suspension beimpft, gut gemischt, anschließend unter sterilen Bedingungen je 20 ml hiervon in Petrischalen gegossen und erstarren lassen. Pro Platte wurden zwei sich gegenüberliegende Löcher (Durchmesser 8 mm) ausgestanzt und je 0,1 ml einer Shampoobasiszusammensetzung gemäß Beispiel 1 von jeweils Climbazole (2,0%), Zinc Pyrithione (2,0%) und Piroctone Olamine (2,0%) bzw. einer Kombination aus Climbazole plus Octopirox (jeweils 2,0% und 0,5%) bzw einer Kombination aus Climbazole (0,5%) plus Zinc Pyrithione (1,0%) plus Piroctone Olamine (0,5%) einpipettiert. Die so beschickten Platten wurden bei 32°C für 48 Stunden mit nach oben gerichteten Deckel bebrütet und anschließend die Durchmesser der Hemmhöfe ausgemessen.

Die folgende Tabelle zeigt die nach dem vorstehenden Verfahren erhaltenen Hemmhofdurchmesser in mm (Lochdurchmesser 8 mm) nach Plattendiffusionstest mit den sieben verschiedenen Stämmen von *Pityrosporum ovale* (*Malassezia furfur*) CBS6000 und DSM6170. Die Zahlenangaben entsprechen den Durchmessern der Hemmhöfe in Millimeter.

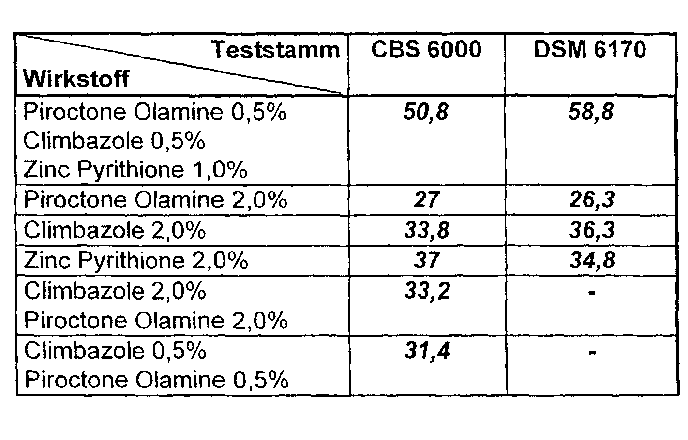

Aus diesen Ergebnissen geht deutlich hervor, dass die erfindungsgemäße Kombination aus Climbazole plus Zinc Pyrithione plus Piroctone Olamine gegenüber einer Kombination aus Climbazole plus Piroctone Olamine und den Einzelwirkstoffen eine deutlich überlegene Wirkung aufweist.

Die erfindungsgemäßen haarkosmetischen Mittel können in allen gebrauchsüblichen Darreichungsformen vorliegen. Beispielsweise als Shampoos (Antischuppen-shampoos), Duschbäder (z.B. Duschgele), Kuren (Antischuppenkuren), Spülungen (Antischuppenspülungen), Haarwässer (Antischuppenhaarwässer), wasserhaltige Haarsprays, Schäume, Gele, Festiger, Deodorantien (z.B. Deo-Stifte), Rasierwässer, Salben oder Cremes.

Bevorzugte Darreichungsform ist ein Shampoo, wobei ganz bevorzugt eine Formulierung auf Basis eines Alkylethersulfates oder eines alkylethersulfathaltigen Tensidgemisches. Als bevorzugte Tenside kommen übliche anionische, amphotere, zwitterionische und/oder nichtionische Tenside in Betracht.

Die betreffenden Zubereitungsformen können in allen für vergleichbare kosmetische Produkte üblichen Behältern abgefüllt und bereitgestellt werden.

Die erfindungsgemäße Kombination der Antischuppenwirkstoffe kann nach bekannten Methoden leicht in jede übliche Basiszusammensetzung für kosmetische oder therapeutische Produkte eingearbeitet werden, wobei eine Wirkstoffmenge zwischen 0,01 und 10,0 Gewichtsprozent (Gew.%) pro Wirkstoff (Climbazole, Zinc Pyrithione, Piroctone Olamine) bezogen auf das Gesamtgewicht der gebrauchsfertigen Zusammensetzung in Betracht kommt, vorzugsweise in einer Menge von 0,05 bis 5,0 Gew.% pro Wirkstoff. Die Menge der Kombination aller drei Wirkstoffe in der gebrauchsfertigen Zusammensetzung kann daher in einem Bereich zwischen 0,03 und 20,0 Gew.%, vorzugsweise zwischen 0,1 und 10,0 Gew.%, insbesondere zwischen 0,5 und 5,0 Gew.% liegen, jeweils bezogen auf das Gesamtgewicht der gebrauchsfertigen Zusammensetzung.

Eine solche Zusammensetzung kann mindestens einen weiteren synthetischen chemischen Antischuppenwirkstoffe und/oder einen solchen auf Naturstoffbasis enthalten. Beispielsweise Salizylsäure, Schwefel, Teerpräparate, Undecensäurederivate, Hinokitiol, Extrakte aus Arnika, Birke, Klettenwurzel, Pappel, Brennessel oder Walnußschale. Die Mengen dieser Wirkstoffe können im Bereich zwischen 0,001 und 10,0 Gew.%, insbesondere zwischen 0,01 und 5,0 Gew.%, ganz besonders zwischen 0,05 und 2,0 Gew.% pro Wirkstoff bezogen auf die gebrauchsfertige Zusammensetzung liegen.

Das erfindungsgemäße Mittel kann alle die üblichen und bekannten Zusatz-, Hilfsund Trägerstoffe enthalten. Beispielsweise Verdickungsmittel wie Bentonit, Fettsäuren, Stärke, Polyacrylsäure und deren Derivate, Zellulosederivate und Alginate; Netzmittel oder Emulgatoren, anionische, kationische, amphotere, zwitterionische oder nichtionogene oberflächenaktive Substanzen (Tenside), beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder ethoxylierte Fettsäureester; Silikontenside oder Silikonöle (beispielsweise Dimethicone), Alkohole wie Äthanol, Propanol, Isopropanol oder Glycerin; Lösungsvermittler, Stabilisatoren, Duftstoffe bzw. Parfüme, Parfümöle, Farbstoffe (zum Beispiel Naturfarbstoffe oder synthetische, direktziehende Farbstoffe) aber auch Anfärbefarbstoffe (beispielsweise Fluorescein-Natriumsalz), haarkonditionierende und haarpflegende Bestandteile bzw. Pflegestoffe wie kationische Polymere, Lanolinderivate, Cholesterin, Panthothensäure und Betain, Konvervierungsmittel, anorganische oder organische Säuren (zum Beispiel Phosphorsäure, Essigsäure, Milchsäure), Basen, Salze (zum Beispiel Natriumchlorid), Puffer (zum Beispiel Natriumcitrat oder Natriumphosphat), Konsistenzgeber (Cellulosederivate), Lichtschutzmittel bzw. UV-Filter, natürliche, teil- oder vollsynthetische Polymere (wie zum Beispiel Chitosan, FMOC-Chitosan, PVP).

Zu den beispielhaft genannten Zusatzstoffen oder Hilfs- und Trägerstoffen, die in dem erfindungsgemäßen Mittel enthalten sein können, zählen beispielsweise Konservierungsstoffe wie Parahydroxybenzoesäure-Ester in einer Menge von 0,05 bis 2,0 Gewichtsprozent), Benzoesäure und Ameisensäure, fungizide und bakteriozide Wirkstoffe (beispielsweise 2,4,4-Trichlor-2-hydroxyphenylether oder Methylchlorisothiazolinon), Pflanzenextrakte (beispielsweise Brennesselextrakt oder Kamillenextrakt in einer Menge von 0,1 bis 2,0 Gewichtsprozent), Lichtschutzmittel (beispielsweise p-Methoxyzimtsäureisoamylester in einer Menge von 0,01 bis 2,0 Gewichtsprozent), Verdickungsmittel (beispielsweise Cellulose und Ester davon in einer Menge von 0,5 bis 3,0 Gewichtsprozent), Haarpflegestoffe (beispielsweise Apfelwachs, Ilexharz, Lanolin, Bienenwachs, Panthenol, Fettalkohole, Fettsäuren, Triglyceride, Ceramide, Betain, Carnitinester, Aminosäuren, Peptide, Proteine, Vitamine, sowie Mischungen davon in einer Menge von 0,1 bis 2,0 Gewichtsprozenten). Als Vitamine kommen beispielsweise Vitamin C, Vitamin B (Biotin), Vitamin E (Tocopherole) jeweils in einer Menge von 0,001 bis 2,0 Gewichtsprozent in Betracht. Als Proteine kann beispielsweise Keratin in einer Menge von 0,1 bis 4,0 Gewichtsprozent Verwendung finden. Als Aminosäuren seien beispielsweise Cystein oder Alanin in einer Menge von 0,01 bis 0,5 Gewichtsprozent genannt. Als Emulgatoren können insbesondere nichtionogene Emulgatoren, beispielsweise Plantacare bzw. Alkyloligoglukosid von Henkel, Düsseldorf, Deutschland, in einer Menge von 0,1 bis 2,0 Gewichtsprozent verwendet werden. Als Lösungsvermittler kommt beispielsweise Cremophore RH 410 bzw. Glycerin-Polyethylenglykoloxystearat von BASF, Ludwigshafen, Deutschland, in einer Menge von 0,1 bis 2,0 Gewichtsprozent in Frage.

Als Silikonöle können die bekannten Produkte verwendet werden, beispielsweise Dimethicone (z.B. Dow Corning 200 Fluids), Phenyltrimethicone (z.B. Abil AV-Typen der Fa. Goldschmidt), Cyclomethicone (z.B. Dow Corning 244 Fluid), Dimethiconol (z.B. Dow Corning 1401), Alkyldimethicone (z.B. Dow Corning 2502 und 2503) oder aminofunktionelle Silikone (z.B. Dow Corning 939 Oder 8220). Die Silikonöle können in Konzentrationen von 0,02 bis 5 Gewichtsprozent, bezogen auf das Gesamtgewicht des Fertigprodukts, verwendet werden.

Die anionischen, kationischen, amphoteren, zwitterionischen oder nichtionischen Tenside können einzeln oder im Gemisch eingesetzt werden.
Als anionische Tenside eignen sich Alkali-, Erdalkali-, Ammonium- oder Alkanolaminsalze von Alkansulfonaten, Alkylsulfonaten und Alkylethersulfaten, die insbesondere 12 bis 18 Kohlenstoffatome im Alkylrest enthalten können, insbesondere die Natrium- oder Triethanolaminsalze von Lauryl- oder Tetradecylethersulfaten.
Betreffend kationische Tenside kommen Fettamine, quartäre Ammoniumverbindungen, beispielsweise Genamin® CTAC bzw. Cetyltrimethylammoniumchlorid (Hoechst, Deutschland), quartäre Verbindungen des Pyridins, des Morpholins oder des Imidazolins in Betracht.
Als nichtionische oberflächenaktive Substanzen können oxethylierte Fettalkohole mit 12 bis 18 Kohlenstoffatomen und mit bis zu 40 Mol Ethylenoxyd pro Mol Fettalkohol eingesetzt werden wie beispielsweise oxethylierter Lauryl-, Tetradecyl-, Cetyl-, Oleyl- oder Stearylalkohol, allein oder im Gemisch, sowie Fettalkohole von oxethyliertem Lanolin oder oxethyliertes Lanolin. Aber auch Fettaminethoxylate, Fettsäurealkanolamide und oxethylierte Sorbitanfettsäureester sind als Tensid oder Tensidgemisch geeignet.
Unter den amphoteren, oberflächenaktiven Substanzen sind beispielsweise die N-Alkylbetaine, die N-Alkylaminobetaine, die N-Alkylsulfobetaine, die N-Alkylaminopropionate, die Alkyldimethylammoniumacetate oder die Fettsäurealkylamidobetaine als geeignete waschaktive Rohstoffe als weitere Komponenten des erfindungsgemäßen haarkosmetischen Mittels zu nennen.
Kationische Polymere sind ebenfalls aus dem Stand der Technik bekannt und kommerziell erhältlich. Es können ein oder mehrere kationische Polymere zugesetzt werden. Geeignet hierfür sind alle kosmetisch akzeptablen, kationisch geladenen Polymere, beispielsweise Luviquat® FC 905 (Copolymer aus Vinylimidazoliummethochloridlvinylpyrrolidon bzw. Polyquaternium-16) von BASF, Ludwigshafen, Deutschland, oder Gafquat® 755 N (Copolymer aus Vinylpyrrolidon/Dimethylaminoethylmethacrylat bzw. Polyquaternium-11) von ISP, New Jersey, U.S.A, oder Abil Quat 3272 (diquarternäres Polydimethylsiloxan bzw. Quaternium-80) von Th. Goldschmidt AG, Essen, Deutschland, oder UcarePolymer JR400 (Polyquaternium-10) von Amerchol, New Jersey, U.S.A., oder Cosmedia Guar C 261 (Hydroxypropyl Guar Hydroxypropyltrimoniumchlorid) von Henkel, Düsseldorf, Deutschland, oder Jaguar C13S (Guar Hydroxypropyltrimoniumchlorid) von Rhöne Poulenc, Frankreich, oder Merquat 550 (Polyquaternium 7) der Fa. Chemviron SA, oder Lamequat L (Lauryldimonium Hydroxypropyl Hydrolyzed Collagen) der Fa. Grünau/Henkel. Die kationischen Polymere können in Konzentrationen von 0,01 bis 3 Gewichtsprozent eingesetzt werden.

Außer den vorstehend genannten Bestandteilen können die erfindungsgemäßen Haarpflegemittel noch weitere Komponenten enthalten, die in Haarpflegemitteln üblicherweise angewendet werden. Hierzu gehören Konsistenzgeber und Verdickungsmittel wie Agar-Agar, Guar-Gum, Alginate und Xanthan-Gum oder Ester aus ethoxylierten Polyolen und Fettsäuren wie Polyglyceryl(2)polyoxyethylen(4)stearat,
Öle und Wachse wie z.B. Triglyceride pflanzlichen oder tierischen Ursprungs, Walrat, Bienenwachs, Montanwachs, Paraffinöle, Vaseline, Wollwachs, Frucht- und andere Pflanzenwachse.

Hinsichtlich der üblichen kosmetischen Zusätze kann überdies auf die dem Fachmann bekannten Publikationen und Monographien verwiesen werden, beispielsweise kann betreffend Shampoozusammensetzungen genannt werden DORNSCH, Andreas, Die kosmetischen Präparate, Verlag chem. Industrie, H. Ziolkowsky KG, Augsburg, Auflage, Band 2, Seiten 212 - 230, 1992.

Das erfindungsgemäße haarkosmetische Mittel kann einen pH-Wert im Bereich zwischen 1,0 und 9,0, vorzugsweise im Bereich zwischen 4,0 und 7,0, ganz besonders im Bereich zwischen 4,5 und 5,6 aufweisen. Gewünschtenfalls kann für die Einstellung eines physiologisch verträglichen pH-Werts eine geeignete Säure verwendet werden. Hierfür eignet sich der Zusatz von Zitronensäure, Weinsäure, Milchsäure, Adipinsäure, Glyoxylsäure, Glukonsäure, Äpfelsäure oder Phosphorsäure.

Es hat sich hierbei herausgestellt, dass die gewünschte Antischuppen-Wirkung in vorteilhafter Weise zu erreichen ist, wenn das erfindungsgemäße Mittel beispielsweise in Form eines Shampoos bei der Haarwäsche mit der Hautoberfläche des Kopfes in Kontakt gebracht wird. Wenn Haarwässer, Kuren oder Spülungen angewendet werden sollen, wird das entsprechende Mittel täglich oder mindestens einmal wöchentlich über einen Zeitraum von Tagen, Wochen oder Monaten oder fortlaufend angewendet. Die Dauer und Häufigkeit der Anwendung kann mit der Art, Bildung und Stärke der Ausprägung der Kopfhautschuppen zusammenhängen.

Die nachfolgenden Beispiele sollen den Gegenstand der Erfindung näher beschreiben. Die angegebenen Zahlenwerte sind Gewichtsprozente bezogen auf 100% Reinsubstanz.

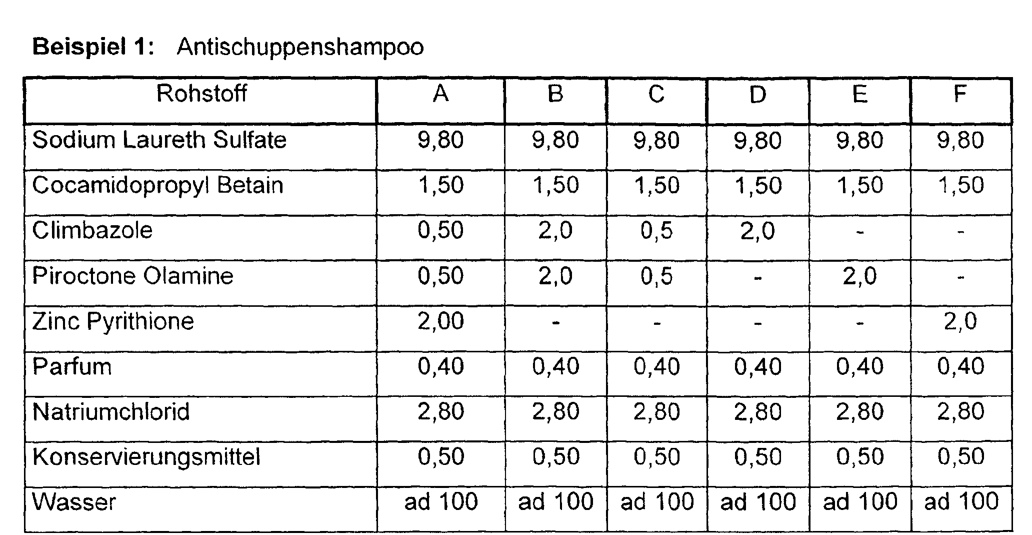

Für die erfindungsgemäße Zusammensetzung A kann gegenüber den Vergleichszusammensetzungen B bis F eine deutlich bessere Antischuppenwirkung erkannt werden.

Die Herstellung der vorstehend genannten Rezeptur kann nach an sich bekannten Verfahren erfolgen. Die Rohstoffe sind allgemein bekannt und sind kommerziell erhältlich.

## Patentansprüche

1. Mittel enthaltend als Kombination die Antischuppenwirkstoffe
a) Climbazole in einer Menge von 0,01 bis 10,0 Gewichtsprozent, vorzugsweise 0,05 bis 5,0 Gewichtsprozent,
b) Zinc Pyrithione in einer Menge von 0,01 bis 10,0 Gewichtsprozent, vorzugsweise 0,05 bis 5,0 Gewichtsprozent,
c) Piroctone Olamine in einer Menge von 0,01 bis 10,0 Gewichtsprozent, vorzugsweise 0,05 bis 5,0 Gewichtsprozent,
jeweils bezogen auf das Gesamtgewicht der gebrauchsfertigen Zusammensetzung, wobei die Menge der Kombination aller drei Wirkstoffe a), b) und c) in der gebrauchsfertigen Zusammensetzung im Bereich zwischen 0,03 und 20,0 Gewichtsprozent, vorzugsweise zwischen 0,1 und 10,0 Gewichtsprozent, noch bevorzugter zwischen 0,5 und 5,0 Gewichtsprozent, liegt, jeweils bezogen auf das Gesamtgewicht der gebrauchsfertigen Zusammensetzung.

2. Mittel nach Anspruch 1, **dadurch gekennzeichnet, dass** es zusätzlich mindestens ein weiteres synthetisches chemisches oder natürliches Antischuppenmittel in einer Menge von 0,001 bis 10,0 Gewichtsprozent, insbesondere zwischen 0,01 und 5,0 Gewichtsprozent, ganz besonders zwischen 0,05 und 2,0 Gewichtsprozent, enthält.

3. Mittel nach Anspruch 2, **dadurch gekennzeichnet, dass** das weitere natürliche oder synthetische chemische oder natürliche Antischuppenmittel ausgewählt ist aus Salizylsäure, Schwefel, Teerpräparaten, Undecensäurederivate, Hinokitiol, Klettenwurzelextrakten, Pappelextrakten, Brennesselextrakten, Walnußschalenextrakten, Birkenextrakten, Weidenrindenextrakten oder Arnikaextrakten.

4. Mittel nach Ansprüchen 1 bis 3, **dadurch gekennzeichnet, dass** es als Shampoo, Duschbad, Duschgel, Gel, Kur, Spülung, Haarwässer, Haarspray, Schaum, Festiger, Deodorant, Rasierwasser vorliegt.

5. Kosmetische Verwendung einer Kombination bestehend aus den Wirkstoffen
a) Climbazole in einer Menge von 0,01 bis 10,0 Gewichtsprozent, vorzugsweise 0,05 bis 5,0 Gewichtsprozent,
b) Zinc Pyrithione in einer Menge von 0,01 bis 10,0 Gewichtsprozent, vorzugsweise 0,05 bis 5,0 Gewichtsprozent,
c) Piroctone Olamine in einer Menge von 0,01 bis 10,0 Gewichtsprozent, vorzugsweise 0,05 bis 5,0 Gewichtsprozent,
jeweils bezogen auf das Gesamtgewicht der gebrauchsfertigen Zusammensetzung, wobei die Menge der Kombination aller drei Wirkstoffe a), b) und c) in der gebrauchsfertigen Zusammensetzung im Bereich zwischen 0,03 und 20,0 Gewichtsprozent, vorzugsweise zwischen 0,1 und 10,0 Gewichtsprozent, noch bevorzugter zwischen 0,5 und 5,0 Gewichtsprozent, liegt, jeweils bezogen auf das Gesamtgewicht der gebrauchsfertigen Zusammensetzung, zur Verhütung oder Verminderung der Schuppenbildung oder zur Beseitigung und Linderung einer bereits bestehenden Schuppenbildung auf behaarten Körperoberflächen.

6. Kosmetische Verwendung nach Anspruch 5, **dadurch gekennzeichnet, dass** die behaarten Körperoberflächen die Kopfhaut, die Bartregion, die Achselhöhlen oder den Intimbereich, umfassen

7. Verwendung einer Kombination aus
a) Climbazole in einer Menge von 0,01 bis 10,0 Gewichtsprozent, vorzugsweise 0,05 bis 5,0 Gewichtsprozent,
b) Zinc Pyrithione in einer Menge von 0,01 bis 10,0 Gewichtsprozent, vorzugsweise 0,05 bis 5,0 Gewichtsprozent,
c) Piroctone Olamine in einer Menge von 0,01 bis 10,0 Gewichtsprozent, vorzugsweise 0,05 bis 5,0 Gewichtsprozent,
jeweils bezogen auf das Gesamtgewicht der gebrauchsfertigen Zusammensetzung, wobei die Menge der Kombination aller drei Wirkstoffe a), b) und c) in der gebrauchsfertigen Zusammensetzung im Bereich zwischen 0,03 und 20,0 Gewichtsprozent, vorzugsweise zwischen 0,1 und 10,0 Gewichtsprozent, noch bevorzugter zwischen 0,5 und 5,0 Gewichtsprozent, liegt, jeweils bezogen auf das Gesamtgewicht der gebrauchsfertigen Zusammensetzung, zur Herstellung einer Präparation für die Behandlung von Schuppen der Haut.

## Claims

1. Composition comprising in combination the active anti-dandruff substances
a) climbazole in an amount of 0.01 to 10.0 weight per cent, preferably 0.05 to 5.0 weight per cent,
b) zinc pyrithione in an amount of 0.01 to 10.0 weight per cent, preferably 0.05 to 5.0 weight per cent,
c) piroctone olamine in an amount of 0.01 to 10.0 weight per cent, preferably 0.05 to 5.0 weight per cent,
based in each case on the overall weight of the ready-to-use composition, the amount of the combination of all three active substances a), b) and c) in the ready-to-use composition being situated in the range between 0.03 and 20.0 weight per cent, preferably between 0.1 and 10.0 weight per cent, more preferably between 0.5 and 5.0 weight per cent, based in each case on the overall weight of the ready-to-use composition.

2. Composition according to Claim 1, **characterized in that** it further comprises at least one additional synthetic chemical or natural anti-dandruff agent in an amount of 0.001 to 10.0 weight per cent, in particular between 0.01 and 5.0 weight per cent, very particularly between 0.05 and 2.0 weight per cent.

3. Composition according to Claim 2, **characterized in that** the additional synthetic chemical or natural anti-dandruff agent is selected from salicylic acid, sulphur, tar preparations, undecenoic acid derivatives, hinokitiol, burdock root extracts, poplar extracts, nettle extracts, walnut shell extracts, birch extracts, willow bark extracts or arnica extracts.

4. Composition according to Claims 1 to 3, **characterized in that** it is in the form of a shampoo, shower bath, shower gel, gel, treatment, rinse, hair lotion, hair spray, foam, hair-setting product, deodorant or shaving lotion.

5. Cosmetic use of a combination composed of the active substances
a) climbazole in an amount of 0.01 to 10.0 weight per cent, preferably 0.05 to 5.0 weight per cent,
b) zinc pyrithione in an amount of 0.01 to 10.0 weight per cent, preferably 0.05 to 5.0 weight per cent,
c) piroctone olamine in an amount of 0.01 to 10.0 weight per cent, preferably 0.05 to 5.0 weight per cent,
based in each case on the overall weight of the ready-to-use composition, the amount of the combination of all three active substances a), b) and c) in the ready-to-use composition being situated in the range between 0.03 and 20.0 weight per cent, preferably between 0.1 and 10.0 weight per cent, more preferably between 0.5 and 5.0 weight per cent, based in each case on the overall weight of the ready-to-use composition, for preventing or lessening the formation of dandruff or for controlling and alleviating existing dandruff formation on body surfaces with hair growth.

6. Cosmetic use according to Claim 5, **characterized in that** the body surfaces with hair growth include the scalp, the beard region, the armpits or the genital area.

7. Use of a combination of
a) climbazole in an amount of 0.01 to 10.0 weight per cent, preferably 0.05 to 5.0 weight per cent,
b) zinc pyrithione in an amount of 0.01 to 10.0 weight per cent, preferably 0.05 to 5.0 weight per cent,
c) piroctone olamine in an amount of 0.01 to 10.0 weight per cent, preferably 0.05 to 5.0 weight per cent,
based in each case on the overall weight of the ready-to-use composition, the amount of the combination of all three active substances a), b) and c) in the ready-to-use composition being situated in the range between 0.03 and 20.0 weight per cent, preferably between 0.1 and 10.0 weight per cent, more preferably between 0.5 and 5.0 weight per cent, based in each case on the overall weight of the ready-to-use composition, for producing a product for treating flaking of the skin.

## Revendications

1. Agent qui contient en combinaison les substances antipelliculaires suivantes :
a) climbazole dans une proportion comprise entre 0,01 et 10 pour cent en poids et de préférence entre 0,05 et 5 pour cent en poids,
b) pyrithione de zinc dans une proportion comprise entre 0,01 et 10 pour cent en poids et de préférence entre 0,05 et 5 pour cent en poids et
c) olamine de piroctone dans une proportion comprise entre 0,01 et 10 pour cent en poids et de préférence entre 0,05 et 5 pour cent en poids,
chaque fois par rapport au poids total de la composition prête à l'emploi, la proportion de la combinaison de toutes les trois substances a), b) et c) dans la composition prête à l'emploi étant dans la plage comprise entre 0,03 et 20 pour cent en poids, de préférence entre 0,1 et 10 pour cent en poids et de façon encore plus préférable entre 0,5 et 5 pour cent en poids, chaque fois par rapport au poids total de la composition prête à l'emploi.

2. Agent selon la revendication 1, **caractérisé en ce qu'**il contient en outre au moins un agent chimique antipelliculaire synthétique ou naturel dans une proportion comprise entre 0,001 et 10 pour cent en poids, en particulier entre 0,01 et 5 pour cent en poids et de façon toute particulière entre 0,05 et 2 pour cent en poids.

3. Agent selon la revendication 2, **caractérisé en ce que** l'autre agent chimique antipelliculaire synthétique ou naturel est sélectionné parmi l'acide salicylique, le soufre, les préparations à base de goudron, les dérivés d'acide undécénique, l'hinokitiol, les extraits de racine de bardane, les extraits de peuplier, les extraits d'ortie, les extraits de noix, les extraits de bouleau, les extraits d'écorce de saule ou les extraits d'arnica.

4. Agent selon les revendications 1 à 3, **caractérisé en ce qu'**il est présente la forme d'un shampoing, d'un produit de bain, d'un gel de douche, d'un gel, d'un traitement, d'un produit de lavage, d'une lotion capillaire, d'une laque pour cheveux, d'une mousse, d'un fixateur, d'un déodorant ou d'une eau de rasage.

5. Utilisation cosmétique d'une combinaison constituée des substances suivantes :
a) climbazole dans une proportion comprise entre 0,01 et 10 pour cent en poids et de préférence entre 0,05 et 5 pour cent en poids,
b) pyrithione de zinc dans une proportion comprise entre 0,01 et 10 pour cent en poids et de préférence entre 0,05 et 5 pour cent en poids et
c) olamine de piroctone dans une proportion comprise entre 0,01 et 10 pour cent en poids et de préférence entre 0,05 et 5 pour cent en poids,
chaque fois par rapport au poids total de la composition prête à l'emploi, la proportion de la combinaison de toutes les trois substances a), b) et c) dans la composition prête à l'emploi étant dans la plage comprise entre 0,03 et 20 pour cent en poids, de préférence entre 0,1 et 10 pour cent en poids et de façon encore plus préférable entre 0,5 et 5 pour cent en poids, chaque fois par rapport au poids total de la composition prête à l'emploi, pour prévenir ou diminuer la formation de pellicules ou pour éliminer ou atténuer des pellicules déjà formées sur des surfaces chevelues ou poilues du corps.

6. Utilisation cosmétique selon la revendication 5, **caractérisée en ce que** les zones chevelues ou poilues du corps comprennent le cuir chevelu, la région de la barbe, le creux des aisselles ou la région intime.

7. Utilisation d'une combinaison de :
a) climbazole dans une proportion comprise entre 0,01 et 10 pour cent en poids et de préférence entre 0,05 et 5 pour cent en poids,
b) pyrithione de zinc dans une proportion comprise entre 0,01 et 10 pour cent en poids et de préférence entre 0,05 et 5 pour cent en poids et
c) olamine de piroctone dans une proportion comprise entre 0,01 et 10 pour cent en poids et de préférence entre 0,05 et 5 pour cent en poids,
chaque fois par rapport au poids total de la composition prête à l'emploi, la proportion de la combinaison de toutes les trois substances a), b) et c) dans la composition prête à l'emploi étant dans la plage comprise entre 0,03 et 20 pour cent en poids, de préférence entre 0,1 et 10 pour cent en poids et de façon encore plus préférable entre 0,5 et 5 pour cent en poids, chaque fois par rapport au poids total de la composition prête à l'emploi, pour préparer un produit destiné au traitement de la desquamation de la peau.
